(19) Europäisches Patentamt
European Patent Office
Office européen des brevets

(11) **EP 3 549 958 A1**

(12) **EUROPEAN PATENT APPLICATION**

(43) Date of publication:
09.10.2019 Bulletin 2019/41

(51) Int Cl.:
***C08B 37/00*** (2006.01)    ***C12P 19/04*** (2006.01)

(21) Application number: 18165788.3

(22) Date of filing: 04.04.2018

(84) Designated Contracting States:
AL AT BE BG CH CY CZ DE DK EE ES FI FR GB GR HR HU IE IS IT LI LT LU LV MC MK MT NL NO PL PT RO RS SE SI SK SM TR
Designated Extension States:
BA ME
Designated Validation States:
KH MA MD TN

(71) Applicant: Clariant International Ltd
4132 Muttenz (CH)

(72) Inventors:
• SIEKER, Tim
 82152 Krailling (DE)
• VERHUELSDONK, Marcus
 82110 Germering (DE)
• ZAVREL, Michael
 82140 Olching (DE)

(74) Representative: Graser, Konstanze
Clariant Produkte (Deutschland) GmbH
IPM / Patent & License Management
Arabellastrasse 4a
81925 München (DE)

(54) **PROCESS FOR THE PURIFICATION OF COMPLEX BIOCOMPOSITIONS**

(57) The present invention relates to a process for the purification of complex biocompositions.

**Description**

The present invention relates to a process for the purification of complex biocompositions.

In recent years, natural alternatives for functional substances such as biopolymers, enzymes, pigments and surfactants became more and more important as environmental protection measures and awareness for the limitation of natural resources increased. Several types of microorganisms are capable of producing a wide variety of valuable compounds, however, purification of such complex compositions and respective isolation of the substance of interest has proven difficult and limits industrial applicability. This applies in particular to compositions comprising polymeric substances such as biopolymers which increase viscosity of the composition and severely influence rheology properties. Castillo *et al.* describe the various obstacles of downstream processing of scleroglucan containing fermentation broth (Castillo et al., Microbial production of scleroglucan and downstream processing, frontiers in microbiology, 15 October 2015).

The inventors of the present invention have set themselves the task to develop a process for the purification of complex biocompositions which can also be applied to composition containing a high content of natural biopolymers, which is economically feasible and environmental friendly and allows the isolation of several substances as structure and features of further components are not affected. The inventive process enables a high recovery yield of a refined-grade biopolymer while preserving all macromolecular features.

This task has been solved by a process comprising the steps

a) Providing a complex biocomposition;

b) Heating of the complex biocomposition to a temperature of from 50 to 140 °C;

c) Separating the heated complex biocomposition into a solid and a liquid phase;

d) Subjecting the liquid phase to an ultrafiltration and thereby obtaining a retentate and a permeate.

In the following, the elements of the present invention will be described in more detail. These elements are listed with specific embodiments, however, it should be understood that they may be combined in any manner and in any number to create additional embodiments.

The variously described examples and embodiments should not be construed to limit the present invention to only the explicitly described embodiments. This description should be understood to support and encompass embodiments which combine the explicitly described embodiments with any number of the disclosed elements. Furthermore, any permutations and combinations of all described elements in this application should be considered disclosed by the description of the present application unless the context indicates otherwise.

Throughout this description and the claims, unless the context requires otherwise, the word "comprise", and variations such as "comprises" and "comprising", will be understood to imply the inclusion of a stated member, integer or step or group of members, integers or steps but not the exclusion of any other member, integer or step or group of members, integers or steps. The terms "a" and "an" and "the" and similar reference used in the context of describing the invention (especially in the context of the claims) are to be construed to cover both the singular and the plural, unless otherwise indicated herein or clearly contradicted by the context. Recitation of ranges of values herein is merely intended to serve as a shorthand method of referring individually to each separate value falling within the range. Unless otherwise indicated herein, each individual value is incorporated into the specification as if it were individually recited herein. All methods described herein can be performed in any suitable order unless otherwise indicated herein or otherwise clearly contradicted by context. The use of any and all examples, or exemplary language (e.g., "such as", "for example", "specific", "specific embodiment"), provided herein is intended merely to better illustrate the invention and does not pose a limitation on the scope of the invention otherwise claimed. No language in the specification should be construed as indicating any non-claimed element essential to the practice of the invention.

Within the present invention the term "complex biocompositions" is to be understood to comprise any composition containing at least two organic substances of different molecular structure and least one cell and/or cell fragment of a microorganism. The inventive process is particularly suitable to be applied to biocompositions containing at least one biopolymer, for example biocompositions containing at least one biopolymer and at least one substance selected from the group consisting of surfactants, enzymes, pigments, organic acids, salts and monomeric, dimeric and/or polymeric sugars. Exemplary biocompositions are (1) biocompositions comprising and least one cell and/or cell fragment of a microorganism and at least one biopolymer, at least one surfactant and at least one pigment; (2) biocompositions comprising at least one biopolymer, at least one surfactant, at least one pigment and at least one organic acid; (3) biocompositions comprising at least one biopolymer, at least one surfactant, at least one pigment and at least one salt. Such biocompositions may be any composition produced by fermentation of a biomass by at least one microorganism wherein the biomass might be of natural origin such as hydrolysate of lignocellulosic biomass or an artificial fermentation medium. The inventive process is particularly suitable for any biocomposition resulting from fermentation of a biomass by at least one fungus belonging to the division of Ascomycota such as biomass having a dry matter content selected from the range of from 0.1 to 0.3 wt.-% and containing less than 0.02 wt.-% of Nitrogen from an organic Nitrogen source. Biocompositions suitable for the inventive process may also contain or may consist of the supernant of a fermentation broth.

Within the present invention the term "biopolymer" is to be understood as any polymer produced by living organisms. The inventive process and system are particularly suitable for biopolymers which show a temperature-independent viscosity within the range from 20 to 100°C or within the range of from 20 to 80°C. The inventive process is particularly suitable for biopolymers containing monomeric units which are covalently bonded such as polymeric carbohydrate structures, rubber, suberin, melanin and lignin. Within an exemplary embodiment, the at least one biopolymer has a molecular weight of at least 0.8 MegaDalton such as scleroglucan, pullulan or beta-glucan. Within another exemplary embodiment, the at least one biopolymer has a molecular weight distribution with a maximum of from 0.5 to 5.0 Million Dalton (MegaDalton) or from 0.75 to 3.5 Million Dalton (MegaDalton) or from 1.0 to 2.0 Million Dalton (MegaDalton). Within an exemplary embodiment the biopolymer has a shear thinning behavior described by a constant $b$ in which $b$ is the gradient between two pairs of values $x1/y1$ and $x2/y2$ where $x$ is the shear rate [s$^{-1}$] in the range of 0.1-100 s$^{-1}$ and $y$ is the dynamic viscosity [mPas] at the given shear rate and at a temperature of the biopolymer between 20°C and 80°C and a concentration between 0.05 and 0.5 wt.-%. The constant $b$ can described by the formula $b= -((lg(y1/y2)/(lg(x1/x2))$. Within a suitable but exemplary embodiment b is selected from the range of from 0.65 to 1.05. Within another exemplary embodiment $b$ is selected from the range of from 0.7 to 1.0. Within another exemplary embodiment $b$ is selected from the range of from 0.75 to 0.9. Within the present invention the term "beta-glucan" is to be understood as referring to any β-D-glucose polysaccharide characterized by the formation of a linear backbone with 1-3 β-glycosidic bonds. Within special embodiments of the present invention, the beta-glucans have a molecular weight distribution with a maximum of from 0.5 to 5.0 Million Dalton (MegaDalton) or from 0.75 to 3.5 Million Dalton (MegaDalton) or from 1.0 to 2.0 Million Dalton (MegaDalton).

Within the present invention, the term "surfactant" is to be understood as comprising any substance which lowers the surface tension (or interfacial tension) between two liquids, between a gas and a liquid, or between a liquid and a solid such as organic compounds which contain both hydrophobic groups and hydrophilic groups (also known as "amphiphilic substances"). Within the present invention exemplary surfactants are aureosurfactin and 3-deoxyaureosurfaction (described e.g. by Kim et al., Aureosurfactin and 3-deoxyaureosurfactin, novel biosurfactants produced by Aureobasidium pullulans, The Journal of Antibiotics (2016) 69, 759-761 L3-GPY).

Within the present invention, the term "pigment" is to be understood as comprising any substance which changes the color of reflected or transmitted light as the result of wavelength-selective absorption. The inventive process is particularly suitable for biocompositions comprising at least one biopigment or biochrome such as melanin.

Within the present invention the term "organic acid" is to be understood as comprising any organic acid known to a person skilled in the art such but not limited to carboxylic acids for example acetic acid, citric acid, formic acid and lactic acid.

Within the present invention the term "salt" is to be understood as comprising any salt containing cations such as but not limited to Na$^+$, K$^+$, Mg$^{2+}$, Ca$^{2+}$, Al$^{3+}$ and/or anions such as but not limited to Cl$^-$, F$^-$, SO4 $^{2-}$, CO3 $^{2-}$.

Within the present invention the term "monomeric, dimeric and/or polymeric sugar" is to be understood as comprising any sugar known to a person skilled in the art as monomeric or polymeric sugar. Monomeric sugars may comprise but are not limited to glucose, fructose, galactose, dimeric sugars may comprise but are not limited to sucrose, lactose, maltose and polymeric sugars may comprise but are not limited to starch, cellobiose, glycogen and starch.

The inventive process is in particular suitable for biocompositions containing 0.1 to 10.0 wt.-% of at least one biopolymer, such as from 0.2 to 7.5 wt.-% or from 0.5 to 5.0 wt.-%. The inventive process is also suitable for biocompositions containing from 0.001 to 2 wt.-% of at least one surfactant, such as from 0.002 to 1.5 wt.-%, 0.003 to 1.2 wt.-%, 0.005 to 1.0 wt.-% or from 0.006 to 0.05 wt.-%. The inventive process is also suitable for biocompositions containing from 0.01 to 3 wt.-% of at least one pigment, such as from 0.02 to 2 wt.-%, 0.03 to 1.5 wt.-%, 0.05 to 1.0 wt.-% or from 0.07 to 0.9 wt.-%. Further suitable biocompositions contain from 0.1 to 10.0 wt.-% of at least one biopolymer, from 0.001 to 2 wt.-% of at least one surfactants and from 0.01 to 3 wt.-% of at least one pigment.

Within step b) of the inventive process, the complex biocomposition is heated to a temperature of from 50 to 140 °C, for example from 60 to 140 °C, or from 70 to 140 °C, such as to a temperature of from 75 to 135 °C or from 80 to 135 °C or from 90 to 135°C. Within a particular suitable embodiment step b) of the inventive process is carried out for a time period of from 5 seconds to 2 hours, such as from 10 seconds to 1 hour or from 15 seconds to 15 minutes. Within a particular suitable embodiment of the inventive process the time period is consisting of a first phase (heating phase) and a second phase (holding phase), for example a heating phase of from 5 seconds to 2 hours and a holding phase of from 1 second to 2 hours. Examples are a heating phase from 10 to 120 seconds to heat the complex biocomposition from a temperature of from 20 to 50 °C to a temperature of from 100 to 140°C and a holding phase of from 1 to 30 seconds.

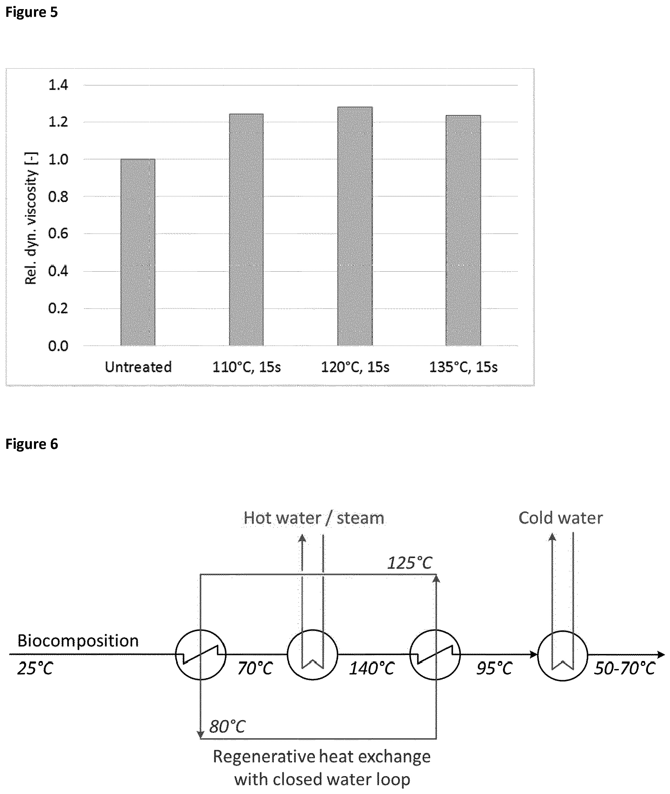

The heating can thereby be carried out by any method known to a person skilled in the art as suitable for the inventive process. A method which is particularly suitable for heating of the complex biocomposition is ultra high temperature heating (also referred to as UHT, ultra heat treatment or ultra pasteurization). The ultra high temperature heating is most advantageously carried out by a two step process including two heating phases as described before wherein it is most advantageous to pre-heat the complex biocomposition to a temperature of from 50 to 70 °C for a time period of from 4 seconds to 4 minutes, and secondly to a temperature of from 70 to 140 °C for a time period of from 1 second to 4 seconds. Particularly suitable combinations are a first heating phase from 5 seconds to 3 minutes to a temperature of from 50 to 70 °C and a second heating phase from 5 seconds to 3 minutes to a temperature of from 80 to 140°C or a first heating phase from 10 seconds to 150 seconds to a temperature of from 60 to 70 °C and a second heating phase from 10 seconds to 150 seconds to a temperature of from 90 to 140°C. A suitable process set-up including an external cooling unit is shown in Fig. 6.

The heating may be carried out directly, where the complex biocomposition is put in a direct contact with the hot steam, or indirectly, where the complex biocomposition and the heating medium remain separated by the equipment's contact surfaces.

Heating the biocomposition according to step b) of the inventive process has the advantage that microbial contamination can be significantly reduced while the valuable components of the complex biocomposition such as the at least one biopolymer or the at least one surfactant or the at least one pigment are still intact.

Within step c) of the inventive process the heated complex biocomposition is separated into a solid and a liquid phase. The separation according to step (c) of the process of the present invention can be carried out by any method known to a person skilled in the art as suitable for the inventive purpose. Within a particularly suitable embodiment, the separation is carried out by solid-liquid-separation, such as filtration, pressing, membrane separation, flotation, precipitation, decantation and centrifugation or combinations thereof. Exemplary separation methods are filter-based solid-liquid separations by use of a filter press. The residues after the filtration should have a minimal solid content of 20 % (wt./wt.), preferably 30 % (wt./wt.), particularly preferred 40 % (wt./wt.) and most preferred 50 % (wt./wt.) solid content. Another method for the separation according to step (c) is centrifugation by e.g. using a decanter. Filtration aids such as diatomaceous earth or perlite can also be added before or during step b) or c) wherein an addition before step c) is most advantageous for the inventive process. Concentrations of from 0.1 wt.-% to 15 wt.-% (weight filtration aid per weight biocomposition), such as from 0.5 wt.-% to 10 wt.-% or from 1 wt.-% to 5 wt.-% of the filtration aid have been found to be of particular advantage.

It is a particular advantage of the inventive process that step c) can be carried out without the addition of a pH agent such as a base or acid.

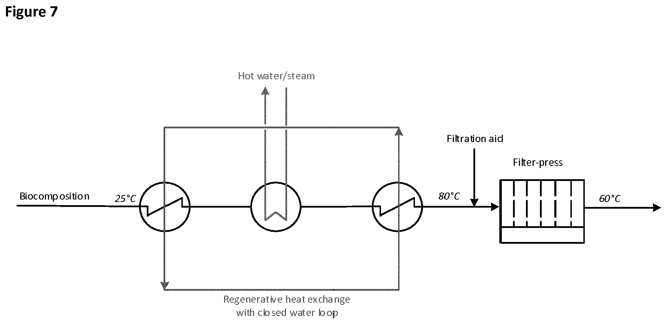

Within a particularly suitable embodiment step c) is carried out at a temperature of from 50 to 80 °C, such as from 55 to 75 °C. It is thereby particular suitable that no separate cooling step is carried out between step b) and step c). This may be achieved by keeping this process (steps b) and c)) within a temperature range of from 50 to 80°C maximum temperature. It is also possible to implement an indirect cooling system (e.g. counterflow heat exchange) by using the residual heat or heat loss within the first heating step of an ultra high temperature heating. An exemplary heat exchanging device is a plate heat exchanger or tube bundle heat exchanger. An exemplary set-up is shown in Fig. 7.

Within another suitable embodiment step c) of the inventive process is carried out for a time period of from 20 seconds to 2 hours, such as from 1 minute to 90 minutes, from 3 minutes to 60 minutes or from 5 minutes to 45 minutes. Within a particular suitable embodiment step c) of the inventive process is carried out at a filtration rate of from 15 to 120 l/(m$^2$h) (m$^2$: filter surface).

Within step d) of the inventive process the liquid phase originating from step c) is subjected to an ultrafiltration to obtain a retentate and a permeate. The ultrafiltration may be carried out by any method known to a person skilled in the art as suitable for the inventive process. The term "ultrafiltration" is well known to a person skilled in the art and constitutes a variety of membrane filtration in which forces like pressure or concentration gradients lead to a separation through a semipermeable membrane. Suspended solids and solutes of high molecular weight are retained in the so-called retentate, while water and low molecular weight solutes pass through the membrane in the permeate (filtrate). Within the present invention a molecular weight cut off MWCO of the ultrafiltration membrane selected from 5 to 500 kDa, such as from 10 to 450 kDa or from 20 to 400 kDa or from 30 to 350 kDa has been found of particular advantage regarding complex biocomposition containing at least one biopolymer. Within a particularly suitable embodiment of the inventive process the MWCO of the ultrafiltration membrane is ¼ to ½ of the molecular weight of the biopolymer. Within a particularly suitable embodiment of the inventive process at least part of the ultrafiltration is carried out by a diafiltration. The term "diafiltration" is well known to a person skilled in the art as a dilution process involving removal or separation of permeable molecules like salts, small proteins, solvents etc.) of a solution based on their molecular size by using micro-molecule permeable filters in order to obtain pure solution. Diafiltration is characterized by adding of an amount of liquid corresponding or equal to the amount removed by filtration. Within another advantageous embodiment of the present invention, the ultrafiltration may be carried out at a temperature of from 5 to 55 °C, wherein a temperature of from 25 to 55 °C and from 40 to 55 °C is also suitable.

Within another advantageous embodiment of the present invention, the inventive process further comprises step e) heating the retentate of step d) to a temperature of from 70 to 140 °C, wherein a temperature of from 75 to 135 °C and from 80 to 130 °C is also suitable.

Within a suitable embodiment step e) of the inventive process is carried out for a time period of from 10 seconds to 2 hours, such as from 15 seconds to 1 hours or from 30 seconds to 30 minutes. The heating according to step e) may also be carried out by a two-step process as described before including a first heating phase for a time from 10 seconds to 150 seconds to heat the retentate to a temperature of from 70 to 90 °C and a second heating phase to heat the retentate for a time period of from 1 second to 150 seconds to a temperature of from 90 to 130 °C.

Within another advantageous embodiment of the present invention, the inventive process further comprises step f1) Precipitation of the liquid phase of step c) or retentate of step d). It is thereby particularly suitable to carry out precipitation by addition of at least one solvent such as ethanol, acetone and isopropanol. Within a particular suitable embodiment of the inventive process step e) is carried out under an inert atmosphere. It is particularly suitable to cool the retentate before or during step e).

Precipitation according to step f1) has the advantage that a solid biopolymer product of high purity can be obtained.

Within another particularly advantageous embodiment of the inventive process step f1) of the inventive process is carried out within a reactor which is not equipped with an internal stirrer or mixing device such as a bubble column or airlift reactor as the at least one biopolymer will stick to the equipment and extraction from the reactor is time consuming and difficult. A gas particularly suitable to be used within these devices is an inert gas.

Within an alternative embodiment of the inventive process, the retentate may also be dried. The inventive process may then further comprise step f2) drying of the retentate. The drying may be carried out by any method known to a person skilled in the art as suitable for the inventive process.

If step f1) is carried out within a bubble column or airlift reactor, the precipitation may be carried out with a specific power input P/V of from 0.02 to 120 W/m$^3$, wherein a specific power input of from 0.05 to 10 W/m$^3$ is also within the scope of the present invention. The specific power input is to be understood as $\frac{P}{V} = \rho * g * u_g,$ wherein g is the gravitational acceleration [m s$^{-2}$], and $u_g$ is the superficial gas velocity is defined as $u_g = \frac{4 * Q_G}{Dr^2 * \pi},$ wherein $Q_G$ is the volumetric gas flow [m$^3$/s], to be understood as the volume of air sparged into the medium, the at least on fungus and the at least one carbon source per second, and Dr is the inner diameter of the vessel.

Within another embodiment the inventive process, further comprises the steps g) to i) to separate at least one surfactant as defined above from the complex biocomposition:

g) contacting the permeate of step d) with at least one organic solvent for a time period of from 5 seconds to 30 minutes;

h) Separating the permeate and at least one organic solvent into two phases of different density;

i) Subjecting the phase of lower density to an evaporation;

j) Obtaining a surfactant as the residual of step i.

The "contacting" may thereby be carried out by any means and measures known to a person skilled in the art as suitable for the inventive process.

Within a particular advantageous embodiment the at least one organic solvent is an organic solvent immiscible with water. Particularly suitable organic solvents are selected from the group consisting of ethylacetate, hexane, butyllactate, methyisobutylketone, heptane and kerosene.

Within another suitable embodiment the at least one organic solvent is added in an amount of from 5 to 30 vol.-%, such as from 8 to 25 vol.-% or from 10 to 22 vol.-%. The contacting is carried out for a time period of from 5 seconds to 30 minutes such as from 10 seconds to 20 minutes, from 15 seconds to 18 minutes or from 30 seconds to 15 minutes.

The separating according to step h) can be carried out by any means or measure known to a person skilled in the art as suitable for the inventive process such as centrifugation or decantation. Suitable separators used within step h) of the inventive process include a centrifugal extractor or decanter and a liquid-liquid separator.

Within step i) of the inventive process, the phase of lower density is subjected to an evaporation to obtain the at least one surfactant. The evaporation may thereby be carried out by any means or measure known to a person skilled in the art as suitable for the inventive process. Within a particularly suitable embodiment the density difference between the phases is at least 5% such as from 5 to 20% or from 5 to 15%. Step i) of the inventive process is most advantageously carried out at a temperature below the boiling point of the organic solvent. Suitable evaporating systems include a distillation column, a thin film evaporator, natural circulation evaporator and wiped film evaporator. Within another suitable embodiment of the inventive process one or more evaporator can be implemented which are particularly suitably implemented in serial circuit. Within another suitable embodiment the retentate is dried after evaporation according to step i). Within this particularly suitable embodiment the inventive process further comprises step j): Obtaining a surfactant as the residual of step i.

Specific embodiments of the present invention

The following specific embodiments define embodiments which are particularly advantageous for the purification of complex biocompositions. These embodiments are not meant to limit the scope of the present application in any respect.

Specific embodiment A

Process for the purification of complex biocompositions comprising the steps

a) Providing a complex biocomposition;

b) Heating of the complex biocomposition to a temperature of from 50 to 140 °C;

c) Separating the heated complex biocomposition into a solid and a liquid phase;

d) Subjecting the liquid phase to an ultrafiltration and thereby obtaining a retentate and a permeate;

wherein no pH agent such as an acid or base is added before or during step c).

Specific embodiment B

Process for the purification of complex biocompositions comprising the steps

a) Providing a complex biocomposition;

b) Heating of the complex biocomposition to a temperature of from 50 to 140 °C;

c) Separating the heated complex biocomposition into a solid and a liquid phase;

d) Subjecting the liquid phase to an ultrafiltration and thereby obtaining a retentate and a permeate;

wherein step b) is carried out at a temperature above 100°C for example at a temperature within the range of from 100°C to 140°C and a separate cooling step is carried out between step b) and step c). In such an embodiment the separating according to step c) is most suitably carried out by use of a filter press.

Specific embodiment C

Process for the purification of complex biocompositions comprising the steps

a) Providing a complex biocomposition;

b) Heating of the complex biocomposition to a temperature of from 50 to 140 °C;

c) Separating the heated complex biocomposition into a solid and a liquid phase;

d) Subjecting the liquid phase to an ultrafiltration and thereby obtaining a retentate and a permeate;

wherein step d) is carried out at a temperature selected from the range of from 5 to 55 °C.

Specific embodiment D

Process for the purification of complex biocompositions comprising the steps

a) Providing a complex biocomposition;

b) Heating of the complex biocomposition to a temperature of from 50 to 140 °C;

c) Separating the heated complex biocomposition into a solid and a liquid phase;

d) Subjecting the liquid phase to an ultrafiltration and thereby obtaining a retentate and a permeate;

f1) Precipitation of the liquid phase of step c), or retentate of step d).

Specific embodiment E

Process for the purification of complex biocompositions comprising the steps

a) Providing a complex biocomposition;

b) Heating of the complex biocomposition to a temperature of from 50 to 140 °C;

c) Separating the heated complex biocomposition into a solid and a liquid phase;

d) Subjecting the liquid phase to an ultrafiltration and thereby obtaining a retentate and a permeate;

f1) Precipitation of the liquid phase of step c), or retentate of step d);

wherein step f1) is carried out in a bubble column reactor.

Specific embodiment F

Process for the purification of complex biocompositions comprising the steps

a) Providing a complex biocomposition;

b) Heating of the complex biocomposition to a temperature of from 50 to 140 °C;

c) Separating the heated complex biocomposition into a solid and a liquid phase;

d) Subjecting the liquid phase to an ultrafiltration and thereby obtaining a retentate and a permeate;

wherein no pH agent such as an acid or base is added before or during step c),
wherein step b) is carried out at a temperature above 100°C for example at a temperature within the range of from 100°C to 140°C and a separate cooling step is carried out between step b) and step c). In such an embodiment the separating according to step c) is most suitably carried out by use of a filter press,
and wherein step d) is carried out at a temperature selected from the range of from 5 to 55 °C.

Specific embodiment G

Process according to specific embodiment F further comprising the steps:

e) Heating the retentate of step d) to a temperature of from 70 to 140 °C;
f1) Precipitation of the liquid phase of step c), or retentate of step d);

wherein step f1) is carried out in a bubble column reactor.

Examples and figures

In the following, the present invention is described by specific examples and figures. The examples and figures are used for illustrating purposes only and do not limit the scope of the present invention.

List of figures

Fig. 1 shows the influence of temperature on filtration

Fig. 2 shows the influence of acid dosing on filtration

Fig. 3 shows the extracted surfactant in the liquid phase

Fig. 4 shows the relative dynamic viscosity at different temperatures

Fig. 5 shows the dynamic viscosity at different UHT treatments

Fig. 6 shows an exemplary set up including a heat exchange device

Fig. 7 shows an exemplary set up for high temperature treatment including a heat exchange device without external cooling and a solid-liquid separation

Example 1

Influence of temperature on filtration

150 kg of a complex biocomposition resulting from fermentation of *Aureobasidium pullulans* (20°C) comprising a beta glucan biopolymer was mixed with a suspension of 30 kg DI-water and 7.5 kg filter aid Dicalite BF (Dicalite Europe nv, Gent, Belgium).
The fermentation was carried out as follows:
100 kg of the following medium was prepared and autoclaved at 121 °C for 20 minutes in a Techfors 150-reactor (Infors AG, Bottmingen, Switzerland):
50 g/kg Sucrose (Südzucker AG), 0.4 g/kg NaNO3 (Sigma Aldrich, Steinheim, Germany), 0.4 g/kg K2HPO4, 0.4 g/kg NaCl (Sigma Aldrich, Steinheim, Germany), 0.4 g/kg MgSO4*7H2O (Sigma Aldrich, Steinheim, Germany), 0.2 g/kg yeast extract (Lallemand, Montreal, Canada), 1 g/kg Antifoam 204 (Sigma Aldrich).
After autoclaving, for the rest of the experiment the temperature was controlled at 26 °C, the pH was adjusted to 4.5 +/- 0.25 using 5 M H2SO4 and 5 M NaOH and controlled to 4.5 +/-0.25 for the remaining fermentation with 5 M NaOH, the medium was stirred at 52 rpm and aerated with 150 L/min at a headspace pressure of 0.2 bar.
When constant conditions were reached, each medium was inoculated to a concentration of 0.019 g/kg CDW of *Aureobasidium pullulans.* The organism was cultivated in the respective medium at the conditions mentioned above for 144 h.
The mixture was heated in a stirred vessel. Temperature (20°C, 50°C, 75°C) and heating time of the complex biocomposition were varied according to table 1. Afterwards the mixture was filtered using a filter press (Netzsch Filtrationstechnik GmbH, Selb, Germany) with pressurized air at 2bar. For the filtration a filter cloth (MarsSyntex PP2442) was used. The produced filtrate mass was recorded versus filtration time. The figure shows the filtration performance which is defined as the produced filtrate mass after 30min filtration time at the respective temperatures divided by the filtrate mass of the reference No. 1, which was carried out at 20°C without heating.

Tab.1 Preparation of filtration samples

| No. | Heating temperature | Holding time at heating temperature | Temperature of filtration |
|-----|---------------------|-------------------------------------|---------------------------|
| 1 | - | - | 20°C |
| 2 | 50 °C | 30 min | 50 °C |
| 3 | 75 °C | 30 min | 75 °C |

(continued)

| No. | Heating temperature | Holding time at heating temperature | Temperature of filtration |
|---|---|---|---|
| 4 | 75 °C | 90 min | 75 °C |
| 5 | 75 °C | 30 min | 20 °C |

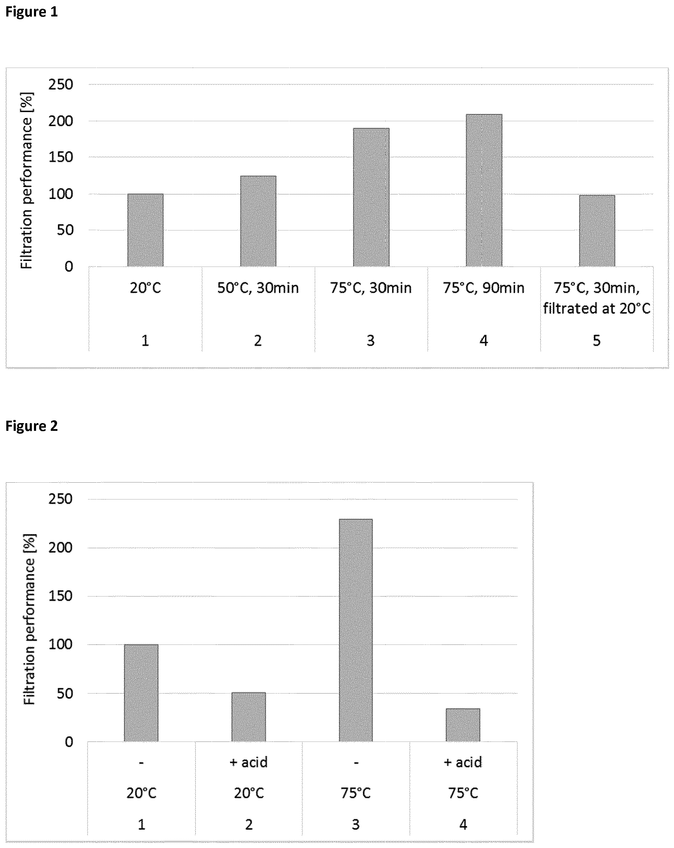

Figure 1 clearly proves that filtration at elevated temperatures improves the filtration performance, which means that the filtration is faster. Comparing experiments No. 3 with No. 5 clearly shows that cooling down to 20°C after the heating step does not improve the filtration.

Example 2

Influence of acid dosing on filtration performance

150 kg of biocomposition (20°C) with the same composition as used in example 1 was mixed with a suspension of 30 kg DI-water and 7.5 kg filter aid Dicalite BF (Dicalite Europe nv, Gent, Belgium). The mixture was heated in a stirred vessel. Temperature (20°C, 75°C) and addition of $HNO_3$ were varied according to table 2. Afterwards the mixture was filtered in a filter press (Netzsch Filtrationstechnik GmbH, Selb, Germany) with a pressurized air at 2 bar. For the filtration a filter cloth (MarsSyntex PP2442) was used. The produced filtrate mass was recorded versus filtration time. Fig. 2 shows the filtration performance which is defined as the produced filtrate mass after 30 min filtration time at the respective temperatures divided by the filtrate mass of the reference No. 1, which was carried out at 20°C without heating.

Tab.2 Preparation of filtration samples with dosing of acid

| No. | Amount of aqueous $HNO_3$(w70% $HNO_3$) | pH-value | Heating temperature | Holding time at heating temperature | Temperature of filtration |
|---|---|---|---|---|---|
| 1 | - | 4.6 | - | - | 20°C |
| 2 | 0.6 L | 2.6 | - | - | 20 °C |
| 3 | - | 4.6 | 75 °C | 30 min | 75 °C |
| 4 | 0.6 L | 2.6 | 75 °C | 30 min | 75 °C |

Figure 2 clearly demonstrates that the best filtration performance is achieved by heating up the complex biocomposition, whereas addition of acid, like proposed in the prior art, even decreases the filtration performance.

Example 3: Obtaining a surfactant-rich liquid product.

For production of the biopolymer the liquid fraction of experiment No.3 of the previous example (75°C, no acid) was concentrated by an ultrafiltration unit with a cut-off of 300 kDa (Synder, LX-3A-2540M) with a concentration factor of three.
Afterwards, 200 mg of solid campher (Alfa Aeser, (1R)-(+)-Campher, 98%) was strewed on the surface of the liquid sample (100 mL) of the obtained permeate. On an aqueous surface without a surfactant the campher moves by transformation of the surface tension. With a surfactant the campher does not move due to the lowered surface tension by the surfactant. For control tap water was used as negative control and a tap water + 0.1wt% cleaning agent containing a surfactant (Ecolab, P3-ultrasil 112) as positive control.

Tab. 3 Test setup: Evidence of surfactant

| No. | Sample | | Campher test |
|---|---|---|---|
| 1 | Water | Negative control | Negative |
| 2 | Water + surfactant | Positive control | Positive |
| 3 | Permeate | Sample | Positive |

Table 3 above clearly proves that by carrying out the ultrafiltration step, a permeate is obtained which contains a surfactant. Since the surfactant was produced by fermentation, the surfactant can be considered as bio-surfactant.

Therefore, it can be demonstrated that the inventive process is able to obtain a surfactant-rich liquid product.

Example 4 Extraction of the surfactant

The permeate of the previous example was mixed with an equal volume ethyl acetate (Sigma Aldrich, Steinheim, Germany) and then the two liquid phases were separated by centrifugation (15 min at 14.000 g). The phase with the lower density was then evaporated in a Heidolph rotary evaporator at 40°C and 150-250 mbar until a solid residual remained in the evaporator. The residual was dissolved in DI-water at pH 5 resulting in a dry matter concentration of 0.1 wt.-%. After shaking a formation of foam was observed.

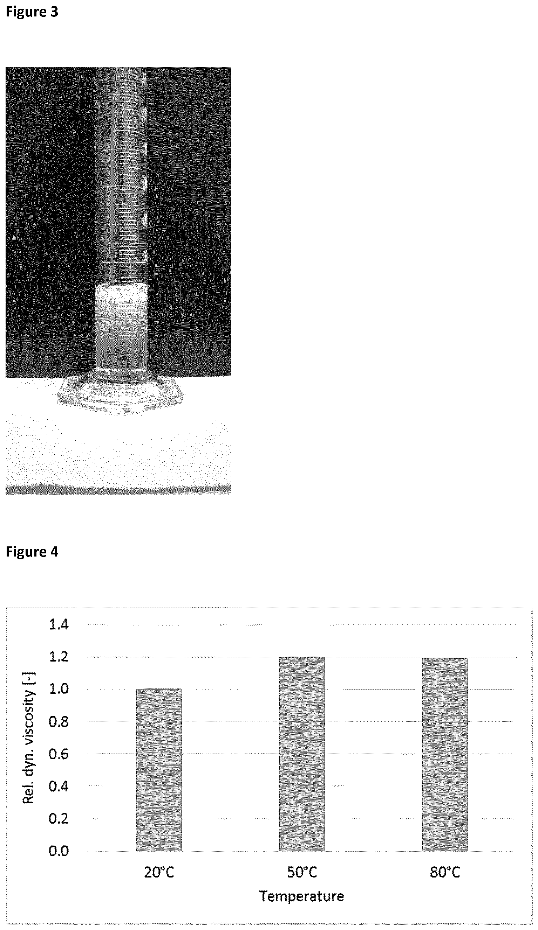

Fig. 3 clearly shows that the invented process is able to produce a surfactant which leads to foam formation in water.

Example 5 Stable viscosity at different temperature

Viscosity of the retentate after ultrafiltration (Synder, LX-3A-2540M, cut-off 300kDa) of the liquid phase of experiment No.3 of the example 2 (75°C, no acid) was measured with a rotational rheometer and coaxial cylinder according to DIN 53019 using a Malvern Kinexus Lab+-rheometer (Malvern Panalytical Ltd., Almelo, Netherlands) at a shear rate of 20 s$^{-1}$ and a temperature of 20 °C, 50°C and 80°C. The figure shows the relative dynamic viscosity which is defined as the viscosity at the measurement temperature divided by the dynamic viscosity at 20°C.

Fig. 4 clearly shows that there is no decrease of the viscosity by increased temperatures. This is in contrast to most biopolymers which typically show a decrease of viscosity by increasing the temperature.

Example 6 Viscosity after UHT treatment

The retentate after ultrafiltration (Synder, LX-3A-2540M, cut-off 300kDa) from the previous example was treated with an UHT (Armfield FT74XTS) at a temperature between 110 and 135 °C with a holding time of 15 s and cooled afterwards to 20°C within 50 s. The samples were measured with a rotational rheometer and coaxial cylinder according to DIN 53019 using a Malvern Kinexus Lab+-rheometer (Malvern Panalytical Ltd., Almelo, Netherlands) at a shear rate of 20 s$^{-1}$ and a temperature of 20 °C. The figure shows the relative dynamic viscosity which is defined as the viscosity with UHT treatment divided by the dynamic viscosity without UHT treatment.

Fig. 5 clearly proves that the UHT treatment does not reduce the viscosity after cooling. Therefore, it can be concluded that the UHT has no negative influence on the biopolymer.

**Claims**

1.  Process for the purification of complex biocompositions comprising the steps

    a) Providing a complex biocomposition;
    b) Heating of the complex biocomposition to a temperature of from 50 to 140 °C;
    c) Separating the heated complex biocomposition into a solid and a liquid phase;
    d) Subjecting the liquid phase to an ultrafiltration and thereby obtaining a retentate and a permeate.

2.  Process according to claim 1, wherein step b) is carried out for a time period of from 5 seconds to 2 hours.

3.  Process according to any of the foregoing claims, wherein the separation according to step c) is carried out by use of a filter press.

4.  Process according to any of the foregoing claims, wherein the molecular weight cut off MWCO of the ultrafiltration membrane is selected from 5 to 500 kDa.

5.  Process according to any of the foregoing claims, wherein at least part of the ultrafiltration is carried out by diafiltration.

6.  Process according to any of the foregoing claims, further comprising step e) Heating the retentate of step d) to a temperature of from 70 to 140 °C.

7.  Process according to claim 6, wherein step e) is carried out for a time period of from 10 seconds to 2 hours.

8. Process according to any of the foregoing claims, further comprising step

   f1) Precipitation of the liquid phase of step c), or retentate of step d).

9. Process according to any of claims 1 to 7, further comprising step

   f2) drying of the retentate.

10. Process according to any of the foregoing claims, wherein the complex biocomposition contains at least one biopolymer.

11. Process according to any of the foregoing claims, wherein at least one filtration aid is added before or during step b) or c).

12. Process according to any of the foregoing claims, wherein step c) is carried out at a temperature of from 50 to 80 °C.

13. Process according to any of claims 1 to 8 or 10 to 12, wherein step f1) is carried out in a bubble column or airlift reactor.

14. Process according to any of the foregoing claims, further comprising steps

   g) contacting the permeate of step d) with at least one organic solvent for a time period of from 5 seconds to 30 minutes;
   h) Separating the permeate and at least one organic solvent into two phases of different density;
   i) Subjecting the phase of lower density to an evaporation;
   j) Obtaining a surfactant as the residual of step i.

**Figure 1**

**Figure 2**

**Figure 3**

**Figure 4**

**Figure 5**

**Figure 6**

**Figure 7**

Hot water/steam

Filtration aid

Filter-press

Biocomposition    25°C    80°C    60°C

Regenerative heat exchange
with closed water loop

Europäisches
Patentamt

European
Patent Office

Office européen
des brevets

# EUROPEAN SEARCH REPORT

Application Number

EP 18 16 5788

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | DAISUKE MURAMATSU ET AL: "Aureobasidium pullulans produced [beta]-glucan is effective to enhance Kurosengoku soybean extract induced Thrombospondin-1 expression", SCIENTIFIC REPORTS, vol. 7, no. 1, 6 June 2017 (2017-06-06), XP055511623, DOI: 10.1038/s41598-017-03053-9 * page 8, paragraph 4 * | 1-14 | INV. C08B37/00 C12P19/04 |
| X | DAISUKE MURAMATSU ET AL: "[beta]-Glucan Derived from Aureobasidium pullulans Is Effective for the Prevention of Influenza in Mice", PLOS ONE, vol. 7, no. 7, 23 July 2012 (2012-07-23), page e41399, XP055511781, DOI: 10.1371/journal.pone.0041399 * page 7, column 1, line 4 - column 2, line 7 * | 1,2,4,8, 10 | |
| X | EP 3 141 607 A1 (CLARIANT INT LTD [CH]) 15 March 2017 (2017-03-15) * claims 1,12 * | 1-14 | TECHNICAL FIELDS SEARCHED (IPC) C08B C12P |
| X | EP 3 141 608 A1 (CLARIANT INT LTD [CH]) 15 March 2017 (2017-03-15) * see also the example; paragraph [0047] - paragraph [0050] * * paragraph [0065] * | 1-14 | |

-/--

The present search report has been drawn up for all claims

| Place of search | Date of completion of the search | Examiner |
|---|---|---|
| Munich | 12 October 2018 | Zellner, Armin |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

**EUROPEAN SEARCH REPORT**

Application Number

EP 18 16 5788

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A,D | JONG-SHIK KIM ET AL: "Aureosurfactin and 3-deoxyaureosurfactin, novel biosurfactants produced by Aureobasidium pullulans L3-GPY", THE JOURNAL OF ANTIBIOTICS, vol. 69, no. 10, 13 January 2016 (2016-01-13), pages 759-761, XP055511626, GB ISSN: 0021-8820, DOI: 10.1038/ja.2015.141 * the whole document * | 1-14 | |
| A | HIROSHI YAMASAKI ET AL: "Characteristics of cross-flow filtration of pullulan broth", APPLIED MICROBIOLOGY AND BIOTECHNOLOGY, SPRINGER, BERLIN, DE, vol. 39, no. 1, 1 April 1993 (1993-04-01), pages 26-30, XP035173231, ISSN: 1432-0614, DOI: 10.1007/BF00166843 * the whole document * | 1-14 | |

**TECHNICAL FIELDS SEARCHED (IPC)**

The present search report has been drawn up for all claims

| Place of search | Date of completion of the search | Examiner |
|---|---|---|
| Munich | 12 October 2018 | Zellner, Armin |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 18 16 5788

This annex lists the patent family members relating to the patent documents cited in the above-mentioned European search report.
The members are as contained in the European Patent Office EDP file on
The European Patent Office is in no way liable for these particulars which are merely given for the purpose of information.

12-10-2018

| Patent document cited in search report | | | Publication date | Patent family member(s) | | Publication date |
|---|---|---|---|---|---|---|
| EP 3141607 | A1 | | 15-03-2017 | AR | 105963 A1 | 29-11-2017 |
| | | | | AU | 2016320216 A1 | 29-03-2018 |
| | | | | CA | 2998283 A1 | 16-03-2017 |
| | | | | CN | 108026554 A | 11-05-2018 |
| | | | | EA | 201890710 A1 | 28-09-2018 |
| | | | | EP | 3141607 A1 | 15-03-2017 |
| | | | | EP | 3347481 A1 | 18-07-2018 |
| | | | | WO | 2017042019 A1 | 16-03-2017 |
| EP 3141608 | A1 | | 15-03-2017 | AR | 105964 A1 | 29-11-2017 |
| | | | | AU | 2016318551 A1 | 29-03-2018 |
| | | | | CA | 2998282 A1 | 16-03-2017 |
| | | | | CN | 108026553 A | 11-05-2018 |
| | | | | EA | 201890708 A1 | 31-08-2018 |
| | | | | EP | 3141608 A1 | 15-03-2017 |
| | | | | EP | 3347480 A1 | 18-07-2018 |
| | | | | US | 2018274000 A1 | 27-09-2018 |
| | | | | WO | 2017042018 A1 | 16-03-2017 |

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **CASTILLO et al.** Microbial production of scleroglucan and downstream processing, frontiers. *microbiology,* 15 October 2015

- **KIM et al.** Aureosurfactin and 3-deoxyaureosurfactin, novel biosurfactants produced by Aureobasidium pullulans. *The Journal of Antibiotics,* 2016, vol. 69, 759-761